# EUROPEAN PATENT APPLICATION

(11) **EP 2 565 628 A1**
(43) Date of publication of application: **06.03.2013**
(21) Application number: 12182520.2
(22) Date of filing: 31.08.2012
(51) Int. Cl.: G01N 21/64

(54) **Nucleic acid detection apparatus, method and program**

(30) Priority: 02.09.2011 JP 2011191947; 02.09.2011 JP 2011191948; 25.07.2012 JP 2012164891
(71) Applicant: Arkray, Inc., Minami-ku Kyoto-shi Kyoto 601-8045 (JP)
(72) Inventor: Hirano, Katsuyasu, Kyoto, Kyoto 602-0008 (JP)
(74) Representative: MacDougall, Alan John Shaw

(57) **Abstract**

Setting an appropriate amplification factor for detecting with good precision the amount of target nucleic acid that has been amplified or melted. A light reception section 14 that receives fluorescence emitted according to the amount of target nucleic acid that has been amplified by PCR due to a light source 12 illuminating excitation light onto a reaction liquid. Electrical signals from the light reception section 14 whose level depends on the received fluorescence intensity are amplified by plural amplification circuits 16a to 16n having different amplification factors from each other. A multiplexor 18 selects an electrical signal amplified with an amplification factor (for example 1 times) in an initial stage of an amplification reaction and detects a fluorescence value for that cycle. A CPU 30 acquires the largest fluorescence value in the initial stage and determines the amplification factor for a corrected stage to be (apparatus detection threshold value - margin)/ largest value of fluorescence value in initial stage, and inputs a selection signal to the multiplexor 18 to select the electrical signal amplified by the determined amplification factor. In the corrected stage the electrical signal amplified with the determined amplification factor is selected and fluorescence values detected.

## Description

### BACKGROUND OF THE INVENTION

### Field of the Invention

The present invention is related to a nucleic acid detection apparatus, method and program.

### Description of the Related Art

When monitoring the amount of an amplified or melted target nucleic acid in amplification methods such as real time polymerase chain reaction (PCR) or melting point temperature measurements, a fluorescence intensity according to the amount of the amplified or melted target nucleic acid is converted into an electrical signal, amplified by a specific amplification factor and acquired. The amplification factor is generally fixed, and the fluorescence intensity to be obtained is controlled by the amount of a reagent added by a user.

Fluorescence detection apparatuses are proposed wherein detection conditions such as a photoelectric amplification factor are set based on data from a subject during detection of the fluorescence emitted from the subject (see for example Japanese Patent Application Laid-Open (JP-A) No. 2009-8603).

Nucleic acid detectors are also proposed wherein post-correction amplification factor D/A values, for amplifying a default correspondence received light signal A/D value up to a target A/D value, are determined in advance for each of plural detection portions, based on default correspondence received light signal A/D values for corresponding target A/D values determined in advance as common values for the plural detection portions against received signals output from the detection portion prior to amplification of the target nucleic acid (see for example JP-A No. 2005-233938).

Light detectors are also proposed wherein plural outputs from an amplifier having different amplification factors to each other are A/D converted, a signal is selected therefrom that is not saturated and that has the highest amplification factor, and processing is performed on the selected signal (see for example JP-A No. 10-96666).

### SUMMARY OF THE INVENTION

However, the technology of JP-A No. 2009-8603 corrects detection data from outside a permitted range to derive a fluorescence characteristic value by employing detection data with different detection conditions (for example, photoelectric conversion amplification factor). The technology of JP-A No. 2005-233938 also reduces the variation between the respective received light levels of the plural detection portions. The technologies of JP-A No. 2009-8603 and JP-A No. 2005-233938 therefore have the problem that appropriate amplification factors cannot be set to detect the amount of amplified or melted target nucleic acid with good precision.

In the technology of JP-A No. 10-96666 plural outputs of different amplification factors are obtained for all the data from the start of measurement to the end of measurement. The technology of JP-A No. 10-96666 therefore has the problem that the required memory capacity increases when measurements are taken over a long period of time, or when a wide range of amplification factors need to be set due to the level of acquired signals being difficult to predict.

In order to address the above issues, an object of at least some embodiments is to provide a nucleic acid detection apparatus, method and program capable of setting an appropriate amplification factor to detect the amount of amplified or melted target nucleic acid with good precision.

In order to achieve the above objective a nucleic acid detection apparatus of a first aspect of the present invention is configured including: a detection unit that detects an amount of an amplified or melted target nucleic acid at plural points in time in an amplification reaction or melting temperature measurement of the target nucleic acid, by detection using an electrical signal whose level depends on light intensity emitted according to the amount of the target nucleic acid; an amplification unit that amplifies an electrical signal detected by the detection unit with a specific amplification factor; and a control unit that, based on an electrical signal detected by the detection unit in an initial stage of the amplification reaction or the melting temperature measurement and based on an apparatus detection threshold value, effects control so as to change the amplification factor of an electrical signal detected during the amplification reaction or the melting temperature measurement after the initial stage.

According to the nucleic acid detection apparatus of the first aspect of the present invention the detection unit detects the amount of the amplified or melted target nucleic acid at plural points in time in the amplification reaction or melting temperature measurement of the target nucleic acid, by detection using an electrical signal whose level depends on light intensity emitted according to the amount of the target nucleic acid. The amplification unit amplifies an electrical signal detected by the detection unit with the specific amplification factor. The control unit then, based on an electrical signal detected by the detection unit in the initial stage of the amplification reaction or the melting temperature measurement and based on the apparatus detection threshold value, effects control so as to change the amplification factor of an electrical signal detected during the amplification reaction or the melting temperature measurement after the initial stage.

The amplification factor for the amplification reaction or the melting temperature measurement after the initial stage is accordingly determined based on the electrical signal detected in the initial stage of the amplification reaction or the melting temperature measurement and based on the apparatus detection threshold value. There is hence no need to output the all of the data with plural different amplification factors, enabling an appropriate amplification factor to be set to detect the amount of the amplified or melted target nucleic acid with good precision while still reducing the memory capacity required.

The first aspect of the present invention may be configured such that the detection unit detects an electrical signal whose level decreases as the amplification reaction or the melting temperature measurement proceeds.

The first aspect of the present invention may also be configured such that a probe capable of hybridizing to a target sequence region of the target nucleic acid is employed in the amplification reaction or the melting temperature measurement. The probe can be configured to emit fluorescence when not hybridized to the target sequence region and have reduced fluorescence intensity when hybridized to the target sequence region.

The first aspect of the present invention may also be configured such that the control unit performs at least one of the following controls when the level of the electrical signal detected by the detection unit in the initial stage exceeds a predetermined range: control to provide notification that an abnormality has occurred in the amplification reaction or the melting temperature measurement; or control to stop the amplification reaction or the melting temperature measurement or control to change the amplification factor.

In order to achieve the above objective a nucleic acid detection apparatus of a second aspect of the present invention is configured including: a detection unit that detects an amount of an amplified or melted target nucleic acid at a plurality of points in time in an amplification reaction or melting temperature measurement of the target nucleic acid, by detection using an electrical signal whose level depends on light intensity emitted according to the amount of the target nucleic acid; an amplification unit that amplifies an electrical signal detected by the detection unit with plural different amplification factors; and a control unit that effects control at each of the plurality of points in time to switch an amplification factor of the amplification unit so as to acquire plural electrical signals amplified by the respective plural different amplification factors.

According to the nucleic acid detection apparatus of the second aspect of the present invention the detection unit detects the amount of the amplified or melted target nucleic acid at a plurality of points in time in the amplification reaction or melting temperature measurement of the target nucleic acid by detecting the electrical signal whose level depends on light intensity emitted according to the amount of the target nucleic acid. The amplification unit amplifies an electrical signal detected by the detection unit with plural different amplification factors. The control unit then effects control, at each of the plurality of points in time, to switch an amplification factor of the amplification unit so as to acquire plural electrical signals amplified by the respective plural different amplification factors.

Acquiring the plural electrical signals amplified with the plural different amplification factors accordingly enables a wide range of amplification factors to be accommodated. Switching the amplification factor and acquiring the signal at each of the points in time, for example by switching so as to acquire only the electrical signals amplified by the amplification factors required, enables flexible amplification factor switching to be performed. Consequently, an appropriate amplification factor can be set for detecting the amount of target nucleic acid with good precision even in cases where it is difficult to predict the level of light intensity for detection.

The second aspect of the present invention may also be configured such that the control unit effects control so as to store a electrical signal that is at a level of an apparatus detection threshold value or lower from among plural acquired electrical signals. The memory capacity required can thereby be effectively reduced.

The second aspect of the present invention may also be configured such that the control unit effefts control so as to display an electrical signal that is at a level of the apparatus detection threshold value or lower from among the plural acquired electrical signals, and to display the level at each point in time of electrical signals amplified with an amplification factor corresponding to an electrical signal with the largest value at the current point in time. Display can accordingly be performed utilizing the gradations currently capable of being displayed on the apparatus to the greatest extent.

The second aspect of the present invention may also be configured such that the control unit effects control such that any electrical signal amplified with an amplification factor corresponding to an electrical signal that has exceeded the apparatus detection threshold value is not acquired at subsequent points in time. A reduction can thereby be achieved in the memory capacity required.

The second aspect of the present invention may also be configured such that the control unit performs at least one of the following controls when all of the plural acquired electrical signals have exceeded the apparatus detection threshold value: control to notify that an abnormality has occurred in the amplification reaction or the melting temperature measurement; or control to stop the amplification reaction or the melting temperature measurement.

An example of the target nucleic acid amplification reaction in the first and second aspects of the present invention comprises real-time PCR.

A nucleic acid detection method of a third aspect of the present invention is a nucleic acid detection method including: detecting an amount of an amplified or melted target nucleic acid at plural points in time in an amplification reaction or melting temperature measurement of the target nucleic acid by detection using an electrical signal whose level depends on light intensity emitted according to the amount of the target nucleic acid; amplifying a detected electrical signal with a specific amplification factor; and based on an electrical signal detected in an initial stage of the amplification reaction or the melting temperature measurement and based on an apparatus detection threshold value, changing the amplification factor of an electrical signal detected during the amplification reaction or the melting temperature measurement after the initial stage.

A nucleic acid detection method of a fourth aspect of the present invention is a nucleic acid detection method including: detecting an amount of an amplified or melted target nucleic acid, at a plurality of points in time in an amplification reaction or melting temperature measurement of the target nucleic acid, by detection using an electrical signal whose level depends on light intensity emitted according to the amount of the target nucleic acid; amplifying a detected electrical signal with plural different amplification factors; and at each of the plurality of points in time, switching an amplification factor of an amplification unit for amplifying the detected electrical signal such that plural electrical signals amplified respectively by the plural different amplification factors are acquired.

A nucleic acid detection program of a fifth aspect of the present invention is a program that causes a computer to function as a control unit for a system in which an amplification factor of an amplification unit is a specific amplification factor for amplifying an electrical signal detected by a detection unit, that detects an amount of an amplified or melted target nucleic acid, at plural points in time in an amplification reaction or melting temperature measurement of the target nucleic acid by detection using an electrical signal whose level depends on light intensity emitted according to the amount of the target nucleic acid, wherein, based on an electrical signal detected by the detection unit in an initial stage of the amplification reaction or the melting temperature measurement and based on an apparatus detection threshold value, the control unit effects control to so as to change the amplification factor of an electrical signal detected during the amplification reaction or the melting temperature measurement after the initial stage of the amplification reaction or the melting temperature measurement.

A nucleic acid detection program of a sixth aspect of the present invention is a program that causes a computer to function as a control unit that effects control so as to switch the amplification factor of an amplification unit employing plural different amplification factors to amplify electrical signals detected by a detection unit, that detects an amount of an amplified or melted target nucleic acid at, a plurality of points in time in an amplification reaction or melting temperature measurement of the target nucleic acid, by detection using an electrical signal whose level depends on light intensity emitted according to the amount of the target nucleic acid, so as to acquire plural electrical signals respectively amplified by the plural different amplification factors.

### Advantageous Effects

As explained above, according to the nucleic acid detection apparatus, method and program of the present invention the advantageous effect is obtained of being able to set an appropriate amplification factor to detect the amount of amplified or melted target nucleic acid with good precision.

### BRIEF DESCRIPTION OF THE DRAWINGS

Exemplary embodiments of the present invention will be described in detail based on the following figures, wherein:
Fig. 1 is a block diagram illustrating a configuration of a nucleic acid detection apparatus of the present exemplary embodiment;
Fig. 2 is a diagram illustrating an example of an amplification circuit;
Fig. 3 is a graph illustrating an example of fluorescence values detected with constant amplification factor;
Fig. 4 is a graph illustrating an example of fluorescence values when the amplification factor in a corrected stage is changed based on the fluorescence values in an initial stage;
Fig. 5 is a graph illustrating the fluorescence values of Fig. 4 for the corrected stage only;
Fig. 6 is a flow chart illustrating the contents of a nucleic acid detection processing routine in a nucleic acid detection apparatus of a first exemplary embodiment;
Fig. 7 is a diagram illustrating an example of a detection results display in the first exemplary embodiment;
Fig. 8 is a diagram illustrating another example of a detection results display in the first exemplary embodiment;
Fig. 9 is a diagram illustrating another example of a detection results display in the first exemplary embodiment;
Fig. 10 is a graph illustrating an example of fluorescence values amplified with 4 steps of amplification factor;
Fig. 11 is flow chart illustrating contents of a nucleic acid detection processing routing in a nucleic acid detection apparatus of a second exemplary embodiment;
Fig. 12 is a diagram illustrating an example of a detection results display in the second exemplary embodiment;
Fig. 13 is a diagram illustrating another example of a detection results display in the second exemplary embodiment; and
Fig. 14 is a diagram illustrating another example of an amplification circuit.

### DETAILED DESCRIPTION OF THE INVENTION

Detailed explanation follows of exemplary embodiments of a nucleic acid detection apparatus of the present invention, with reference to the drawings.

### First Exemplary Embodiment

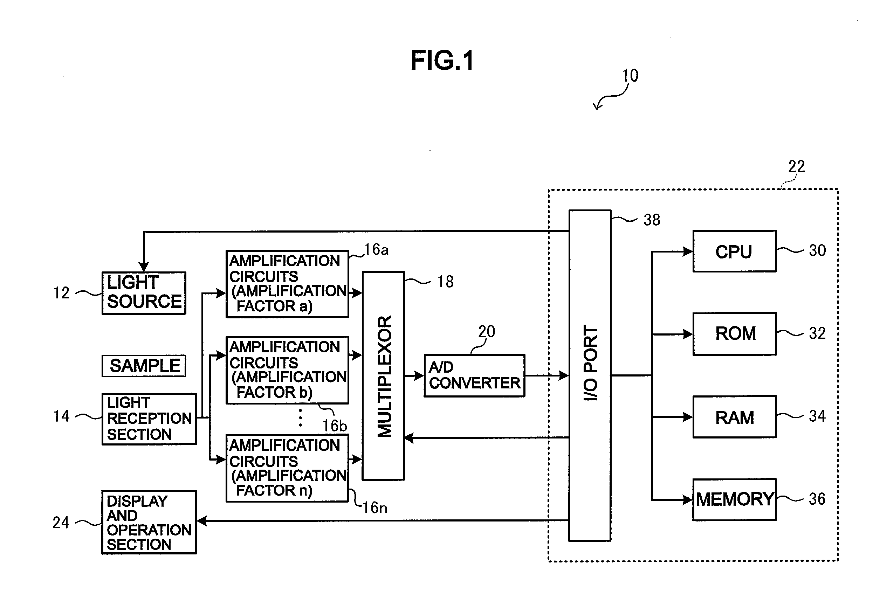

As shown in Fig. 1, a nucleic acid detection apparatus 10 of a first exemplary embodiment is configured including: a light source 12 that is configured for example from LEDs and illuminates a sample with excitation light; a light reception section 14 that receives fluorescence emitted due to excitation light illumination and is configured for example from photodiodes that output an electrical signal at a level that depends on the received light intensity; plural amplification circuits 16a to 16n that amplify the electrical signals output from the light reception section 14 with respective amplification factors; a multiplexor 18 that selects one electrical signal from the amplified electrical signals; an A/D converter 20 that converts the selected analogue electrical signal into a digital signal; a computer 22; and a display and operation section 24 configured for example by a touch panel display into which various data is input by operation and that displays information such as detection results.

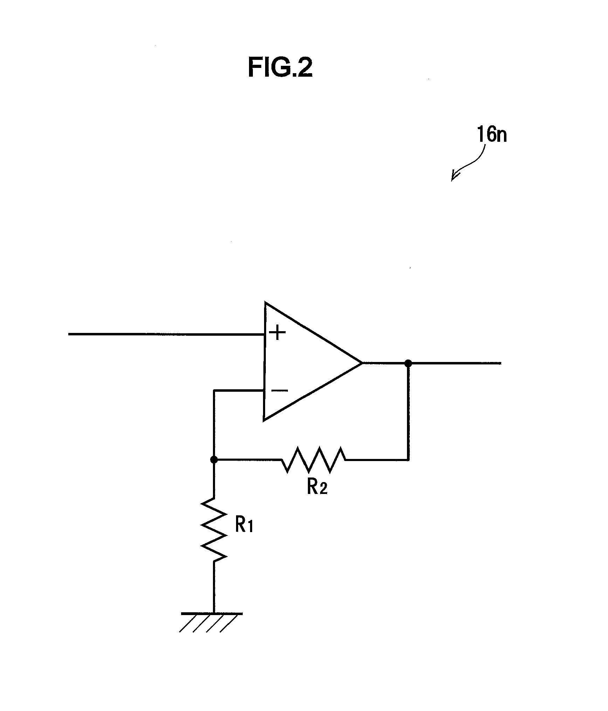

The amplification circuits 16a to 16n may, for example, be configured as inverting amplification circuits as shown in Fig. 2. The amplification factor n of the amplification circuits 16n shown in Fig. 2 is determined as n = 1 + R2/R1. Therefore the resistance values R1 and R2 are set in each of the amplification circuits 16 to achieve the desired values for the amplification factors a, b, and so on to n of each of the amplification circuits 16a, 16b to 16n. Note that the amplification factors a, b, and so on to n of each of the amplification circuits 16a, 16b to 16n are each different values to each other.

The computer 22 is configured including: a CPU 30 that performs overall control of the nucleic acid detection apparatus 10; a ROM 32 serving as a storage medium stored with various programs such as for nucleic acid detection processing, described later; a RAM 34 serving as a work area for temporarily storing data; a memory 36 serving as a storage unit stored with various data; an input-output port (I/O port) 38; and a bus connecting these sections together. A HDD may also be provided.

Explanation follows regarding the principle of the first exemplary embodiment. Explanation here is of a reaction system in which the level of the fluorescence intensity emitted (the electrical signal output from the light reception section 14) falls as the amplification reaction progresses. The electrical signals amplified by the respective amplification circuits 16a to 16n are referred to below as fluorescence values.

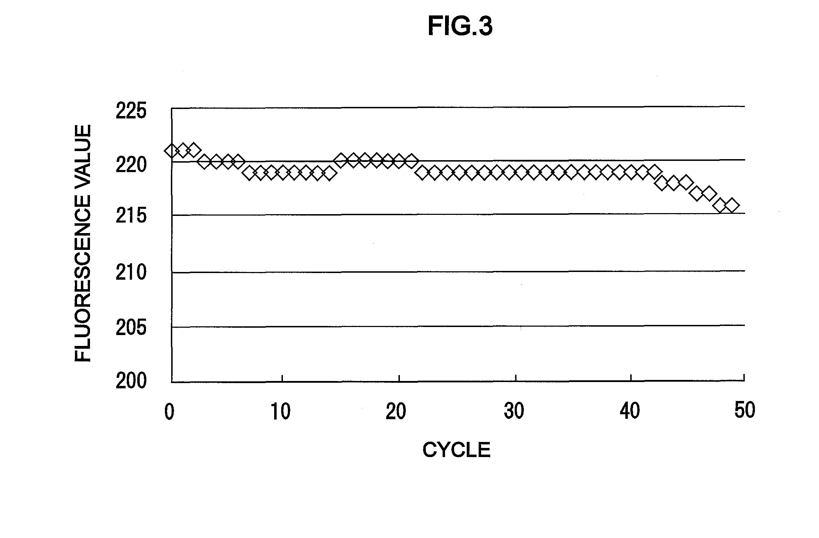

In a reaction system as described above in which the emitted fluorescence intensity falls, Fig. 3 illustrates an example of fluorescence values in a case where the amplification factor is constant over the entire reaction duration of the amplification reaction (all cycles). In the example shown in Fig. 3 the amplification factor is 1 (no amplification). CT (Threshold Cycle) value derivation and the like are performed based on the changes to the thus obtained fluorescence intensities. Often data is employed with a large change in fluorescence value in the latter half portion of the amplification reaction in cases such as CT value derivation. It is therefore desirable to be able to detect the fluorescence values of the latter half portion of the amplification reaction with better precision.

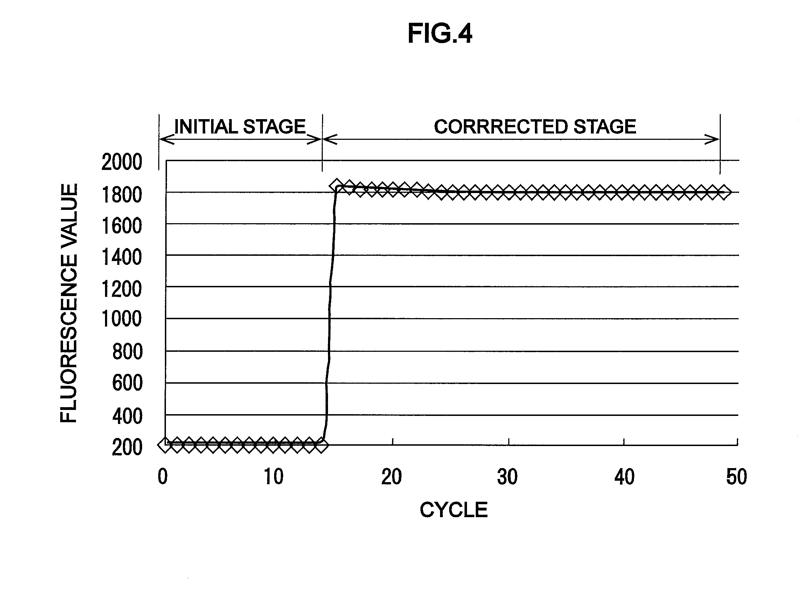

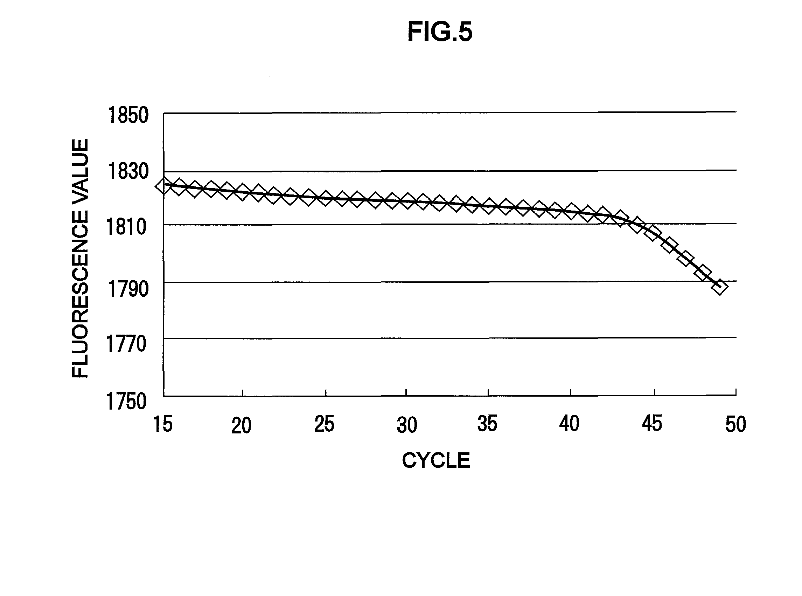

Accordingly, in the nucleic acid detection apparatus 10 of the first exemplary embodiment the amplification factor for the electrical signals detected after an initial stage in the amplification reaction (referred to below as a corrected stage) the amplification factor of the electrical signals for detection is changed to an appropriate value based on the fluorescence values in the initial stage of the amplification reaction. More specifically, the amplification factor in the corrected stage is changed according to the fluorescence values that were detected in the initial stage such that the fluorescence values being detected are expressed using the threshold value of gradations capable of being expressed by the apparatus (detection threshold value) to the greatest extent. For example, the amplification factor can be derived as: detection threshold value/ largest value of fluorescence value in initial stage. Fig. 4 illustrates an example of fluorescence values when the corrected stage amplification factor has been changed based on the fluorescence values in the initial stage. Fig. 5 illustrates an example showing only the corrected stage fluorescence values illustrated in Fig. 4. Due to using the gradations capable of being expressed by the apparatus to the greatest extent, precise changes in fluorescence value can be captured as shown in Fig. 5, enabling fluorescence values to be detected with good precision.

While an example has been explained above in which the amplification factor is computed using the largest value of the initial stage fluorescence values, a value such as the average value of the fluorescence values in the initial stage may be employed therefor. When computing the amplification factor, a specific margin to the apparatus detection threshold value may be provided, and a value may be employed that is slightly lower than the actual detection threshold value. Providing a margin to the detection threshold value enables fluorescence values to be detected without exceeding the detection threshold value even when fluorescence values in the corrected stage are slightly higher than the fluorescence values of the initial stage.

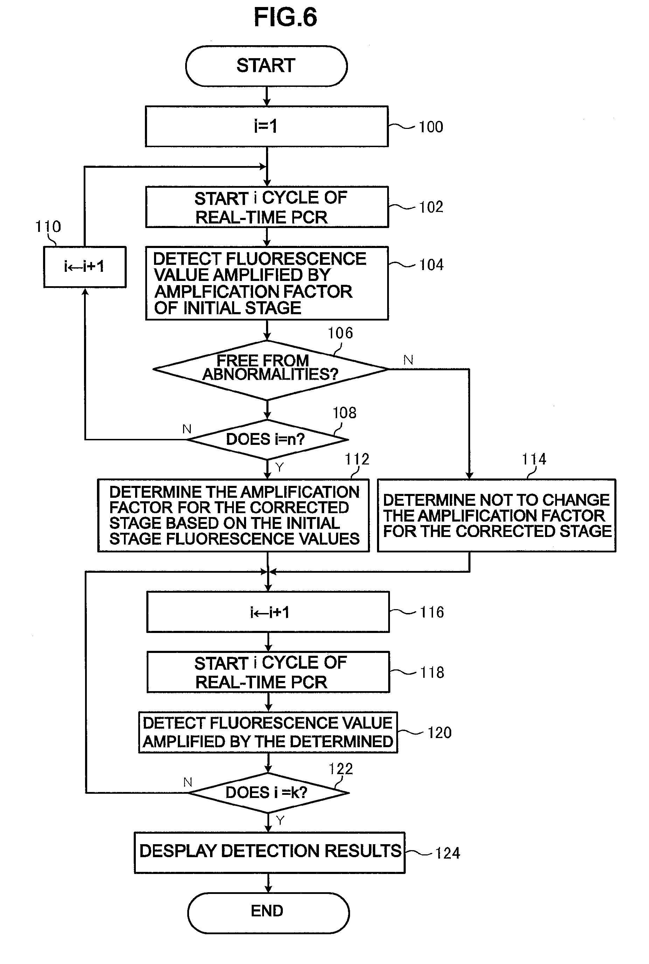

Explanation follows regarding operation of the nucleic acid detection apparatus 10 according to the first exemplary embodiment. Explanation here is of a case in which amplification of target nucleic acid is performed in real-time PCR. First a PCR reaction liquid (sample) including a specimen and a reagent such as a probe, is placed in an analyzing section, not shown in the drawings. The probe employed here is a probe that emits fluorescence when not hybridized to the region of the target sequence of target nucleic acid, and has reduced fluorescence intensity when hybridized to the target sequence region. For example, what is referred to as a fluorescence quenching probe, such as the known guanine quenching probe QProbe (registered trademark) may be employed. Such cases result in a reaction system in which the level of the fluorescence values detected falls as the amplification reaction progresses. The nucleic acid detection processing routine illustrated in Fig. 6 is then executed by the CPU 30 by analysis start instruction from the display and operation section 24.

At step 100 a variable i indicating the number of cycles of real-time PCR is set to 1.

Then at step 102 the i^{th} cycle of real-time PCR is started. In real-time PCR the temperature of the analyzing section is controlled by a temperature control section, not shown in the drawings, and the temperature of the PCR reaction liquid is raised to a first temperature (for example 95°C) and maintained there for a first period of time (for example 60 seconds). In this interval double-stranded DNA is converted into single-stranded DNA. The temperature of the PCR reaction liquid is then lowered to a second temperature (for example 60°C) and maintained there for a second period of time (say 15 seconds). Annealing of the single-stranded DNA and a primer occurs in this period. The temperature of the PCR reaction liquid is then raised to a third temperature (for example 70°C) and maintained there for a third period of time (for example 60 seconds). DNA is synthesized in this period by the action of DNA polymerase. This completes a single cycle.

Then at step 104 the fluorescence value corresponding to the fluorescence intensity emitted in the PCR started at step 102 is detected. More specifically, excitation light is illuminated by the light source 12 onto the PCR reaction liquid placed in the analyzing section. Due to the excitation light, fluorescence is emitted according to the amount of target nucleic acid that has been amplified by PCR and is present in the PCR reaction liquid. When the emitted fluorescence is received by the light reception section 14, the light reception section 14 outputs an electrical signal at a level corresponding to the received fluorescence intensity. The output electrical signal is input respectively to the plural amplification circuits 16a to 16n that have different amplification factors from each other. The electrical signal is amplified here according to the amplification factor of each of the amplification circuits 16a to 16n and then output.

The multiplexor 18 is input with a selection signal from the CPU 30 for selecting an electrical signal that has been amplified by a specific amplification factor. Since the routine is currently in the initial stage of the amplification reaction (cycles 1 to n, for example cycles 1 to 15 out of a total of 50 cycles), the selection signal input to the multiplexor 18 is the selection signal for selecting the electrical signal output from the amplification circuit with amplification factor 1 times. Note that the amplification factor in the initial stage is not limited to 1 times, and, in consideration of the specimen and reagent employed and the apparatus detection threshold value, any value may be employed that ensures the fluorescence values in the initial stage do not exceed the detection threshold value. The selected electrical signal is converted into a digital signal by the A/D converter 20 and input to the computer 22. The level of this electrical signal (the fluorescence value) is stored in the memory 36 against the cycle variable i.

Then at step 106, determination is made as to whether or not the fluorescence value detected at step 104 is abnormal. An appropriate range of fluorescence values for the initial stage is determined in advance based on such factors as the type of specimen and reagent. No abnormality is determined to have occurred as long as the detected fluorescence value falls within the appropriate range, and the routine transitions to step 108. However an abnormality is determined to have occurred when the detected fluorescence value is outside of the appropriate range, and the routine transitions to step 114.

At step 108 whether or not initial stage fluorescence value detection has been completed is determined by determining whether or not the variable i indicating the cycle number has reached n. Processing transitions to step 110 when i ≠ n since the initial stage of fluorescence value detection has not yet been completed. At step 110 the variable i is incremented by 1 and then processing returns to step 102, and processing is repeated for the next cycle of PCR. However processing transitions to step 112 when i = n.

At step 112 the largest value is acquired from the fluorescence values of the initial stage (cycles 1 to n) stored in the memory 36. Then the amplification factor for the corrected stage is determined, for example as (apparatus detection threshold value - margin)/largest value of fluorescence value in initial stage. For example, in a case in which the largest value of acquired initial stage fluorescence values is "220" and the apparatus detection threshold value is "2000" and the margin is "150" then the amplification factor for the corrected stage may be determined as (2000-150)/ 220 = 8.4. The selection signal for selecting the electrical signal amplified by the determined amplification factor is then input to the multiplexor 18. Configuration may be made such that when there is no amplification circuit present with an amplification factor that matches the determined amplification factor, the electrical signal from the amplification circuit having the nearest amplification factor to the determine amplification factor and not exceeding the determined amplification factor is selected.

However when determined at step 106 that the fluorescence value is abnormal and processing has moved to step 114, determination is made not to change the amplification factor for the corrected stage. Since in this example the amplification factor of the initial stage is 1 the amplification factor for the corrected stage is also 1.

Then at step 116 the variable i is incremented by 1 and the processing of the i^{th} cycle of real-time PCR is started at step 118.

Next at step 120 the fluorescence values are detected similarly to in step 104. However, in cases in which processing has transitioned to the current step through step 112, the electrical signal for selecting the amplification factor determined using the initial stage fluorescence values is being input to the multiplexor 18. The electrical signal amplified by the amplification factor determined at step 112 is accordingly selected. However in cases in which processing has transitioned to the current step through step 114, the amplification factor has not been changed and so the electrical signal output from the amplification circuit with amplification factor 1 times remains selected. The selected electrical signal is converted by the A/D converter 20 into a digital signal, input to the computer 22 and stored in the memory 36 against the cycle number i.

Then at step 122 whether or not the total number of cycles have been completed is determined by determining whether or not the variable i indicating the cycle number has reached k. For example k may be set at 50 times. When i ≠ k processing transitions to step 116 since the total number of cycles has not yet been completed, the variable i is incremented by 1 and processing is repeated for the next cycle of PCR. However processing transitions to step 124 when i = k.

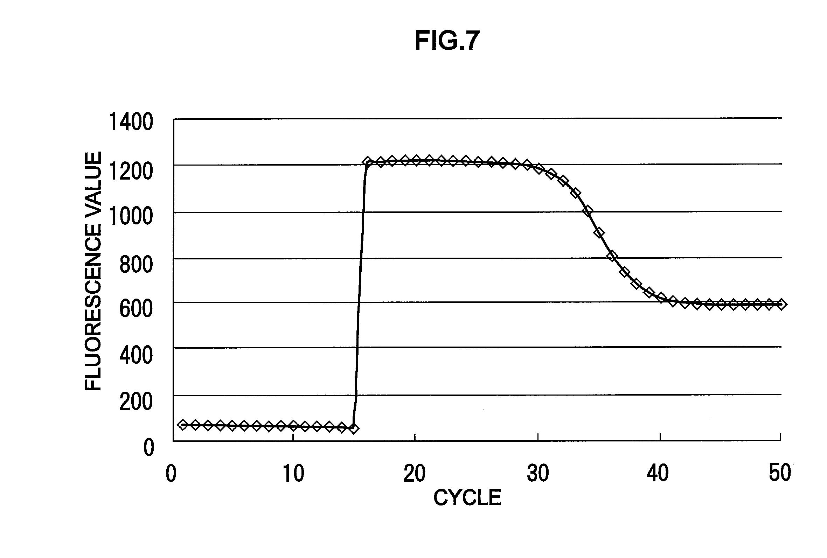

At step 124 the fluorescence values stored in the memory 36 are, for example, plotted as a graph of fluorescence values against cycle number as shown in Fig. 7, and then displayed as detection results on the display and operation section 24. Processing is then ended.

As explained above, according to the nucleic acid detection apparatus of the first exemplary embodiment, the amplification factor for after the initial stage in the amplification reaction (the corrected stage) is determined based on the fluorescence values detected in the initial stage of the amplification reaction and based on the apparatus detection threshold value. The threshold value of gradations capable of being expressed by the apparatus are therefore utilized to the greatest extent and so precise changes in the fluorescence values can be captured, enabling the fluorescence values to be detected with good precision. The amplification factor can be set automatically in this manner.

Utilizing a probe with a high thermal stability enables the probe to be introduced in advance during a PCR reaction and enables a reaction that is reversible with temperature change to be performed repeatedly. Utilizing a quenching probe as in the first exemplary embodiment enables the probe amount to be estimated from the fluorescence values detected in the initial stage of PCR cycles, and enables correction to be made to the light intensity with the optimal gain for subsequent Tm analysis when measuring the reaction liquid of unknown concentration in the PCR processes.

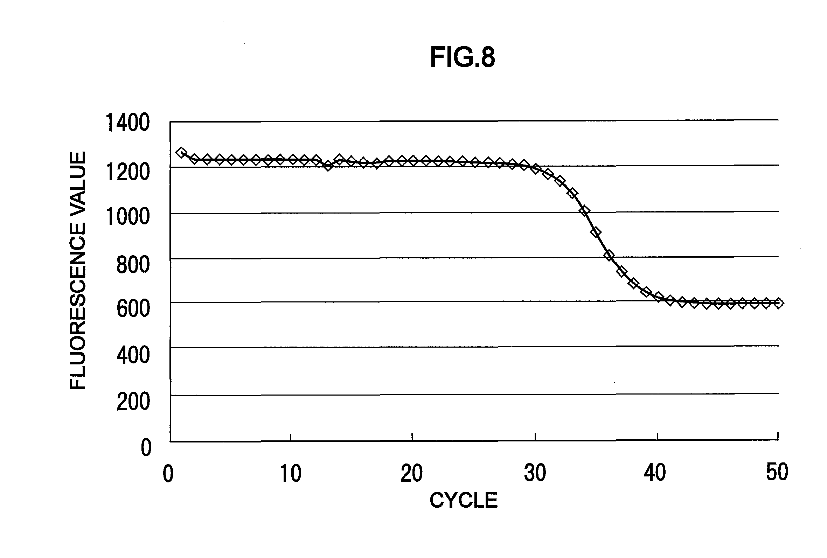

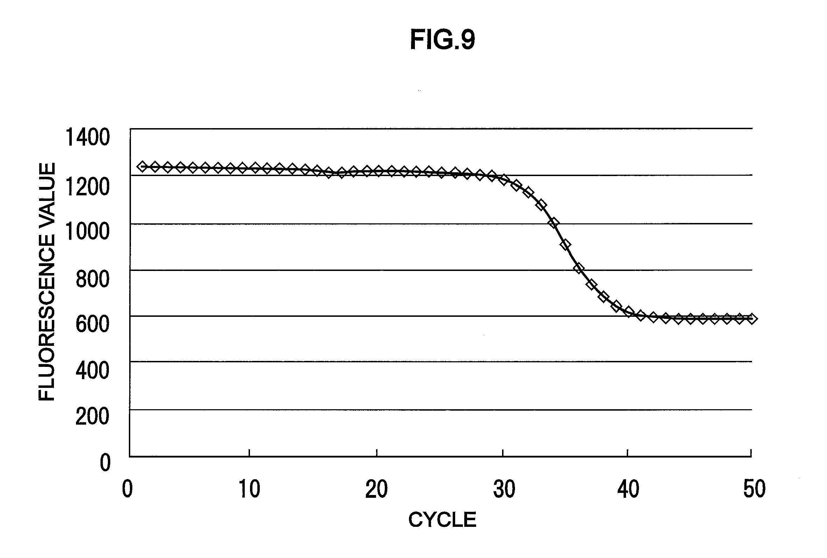

Note that in the first exemplary embodiment explanation has been given of a case in which a graph is displayed with the detected fluorescence values plotted as they are when the detection results are being displayed, however there is no limitation thereto. For example, as shown in Fig. 8, configuration may be made such that the fluorescence values of the initial stage are corrected to give values amplified by the amplification factor for the corrected stage determined at step 112. In the first exemplary embodiment, discrete fluorescence values detected at each cycle are displayed, however, as shown in Fig. 9, correction to give a smooth graph for display may be made by performing smoothing and baseline correction. While explanation has been given in the first exemplary embodiment of a case in which detected results are displayed after completing all the cycles configuration may be made such that the fluorescence values are plotted and displayed after each single cycle is completed.

Explanation has been given of an example in the first exemplary embodiment in which the initial stage is taken as cycles 1 to 15 from a total cycle number of 50 cycles, however the initial stage is appropriately settable for example as 1/2, 1/3 or 1/4 of the total cycle number. Increasing the number of cycles in the initial stage enables a more appropriate amplification factor for the corrected stage to be determined. Reducing the number of cycles in the initial stage leads to a greater number of cycles being required to enable the fluorescence value to be detected with good precision by the appropriately set amplification factor. An appropriate number of cycles for the initial stage is preferably set according to the characteristics of the specimen employed. Alternatively, the initial stage may be defined as, for example, 1/2, 1/3 or 1/4 of the total reaction time, instead of in relation to the number of cycles.

Explanation has been given in the first exemplary embodiment of a case in which control is made such that the amplification factor for the corrected stage is not changed when the fluorescence values detected in the initial stage are determined to be abnormal, however control may be performed to stop the amplification reaction itself in such circumstances. Alternatively configuration may be made such that instead of such control, or in addition to such control, notification is made, such as by displaying a message indicating that abnormal initial stage fluorescence values have occurred on the display and operation section.

Explanation has been given of a case in the first exemplary embodiment in which the amplification factor in the corrected stage is constant, however configuration may be made such that the amplification factor in the corrected stage is switched in plural steps. For example, in a reaction system in which the fluorescence values fall as the amplification reaction progresses, configuration may be made such that the amplification factor is switched to gradually rise as the number of cycles in the corrected stage progresses.

Explanation has been given of a case in the first exemplary embodiment wherein a probe is employed that emits fluorescence when not hybridized to the target sequence region of the target nucleic acid, and has reduced fluorescence intensity when hybridized to the target sequence region, however there is no limitation thereto. For example, a probe may be employed that emits fluorescence when hybridized to the target sequence region of the target nucleic acid and has reduced fluorescence intensity when not hybridized to the target sequence region.

Explanation has been given of a case in the first exemplary embodiment of a reaction system in which the fluorescence values fall as the amplification reaction progresses, however application is possible to a reaction system in which the fluorescence values increase as amplification progresses. In such cases configuration may be made such that the fluorescence values for the corrected stage are estimated from the initial stage fluorescence values based on such factors as the type of specimen, and the amplification factor is determined such that the corrected stage fluorescence values can be expressed by utilizing the apparatus detection threshold values to the greatest extent.

Explanation has been given of a case in the first exemplary embodiment in which the electrical signal amplified with the appropriate amplification factor is selected by a multiplexor from the electrical signals output from the plural amplification circuits having different amplification factors and input to a computer, however there is no limitation thereto. For example, configuration may be made such that each of the electrical signals amplified by the amplification circuits is input to a computer, where the electrical signal amplified by the appropriate amplification factor is selected by a CPU and stored in a memory.

Note that although in the first exemplary embodiment a case of an amplification reaction by real-time PCR has been explained the present invention can also be applied to a melting temperature measurement of target nucleic acid. In such cases a single cycle of the above nucleic acid detection processing routine may be performed for each temperature of the measurement points (for example every 1°C). For example, when melting temperature measurements are executed every 1°C over a range from 0°C to 100°C, the initial stage is defined as 0°C to 10°C and the corrected stage is defined as 11°C to 100°C, wherein configuration may be made such that the amplification factor for the corrected stage is determined based on the fluorescence values detected in the initial stage.

### Second Exemplary Embodiment

Explanation follows regarding a second exemplary embodiment. The configuration of the nucleic acid detection apparatus of the second exemplary embodiment is similar to the configuration of the nucleic acid detection apparatus 10 of the first exemplary embodiment and so the same reference numerals are allocated and detailed explanation thereof is omitted.

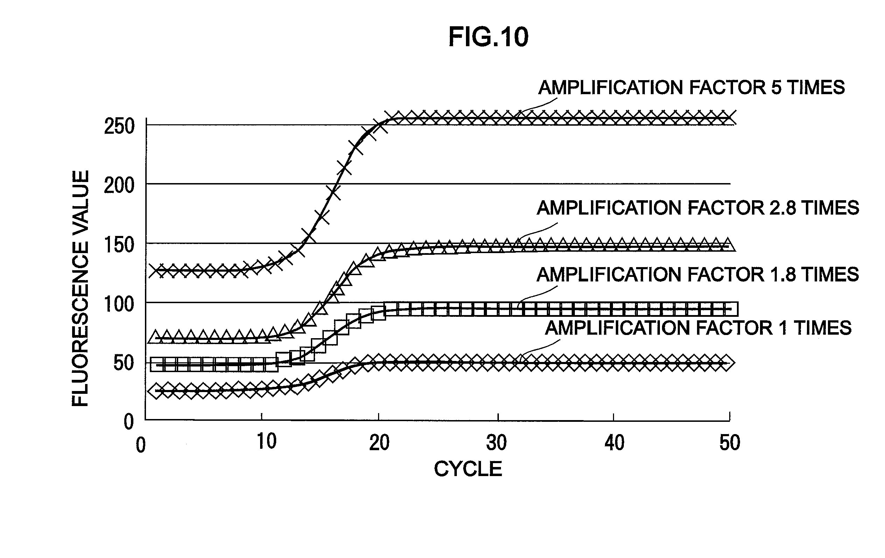

Explanation follows regarding the principle of the second exemplary embodiment. Explanation follows regarding a reaction system in which the level of the fluorescence intensity emitted (the electrical signal output from the light reception section 14) increases as the amplification reaction progresses. Fig. 10 illustrates an example of fluorescence values amplified by 4 steps of amplification factor in a reaction system in which the emitted fluorescence intensity increases. In the example of Fig. 10, the amplification factors are 4 steps of 1 times, 1.8 times, 2.8 times and 5 times. The fluorescence values can be detected with good precision since the threshold value of gradations capable of being expressed by the apparatus (detection threshold values, for example in this case 256) can be more effectively utilized as the amplification factor increases.

However, in the example of Fig. 10, the fluorescence values amplified by the largest amplification factor of the 4 steps (5 times) exceeds the detection threshold value at about the 21^{st} cycle, and correct fluorescence values cannot be taken for subsequent cycles. In particular, it is difficult to set an appropriate amplification factor in advance in cases where changes to the fluorescence values are difficult to predict. Suppose only the amplification factor of 5 times has been set in the above example then normal detection results would not be obtainable.

Therefore, as shown in Fig. 10, a wide range of amplification factors can be accommodated by acquiring each of the fluorescence values amplified by plural amplification factors. However a substantial memory capacity is required when fluorescence values amplified by each of the amplification factors are acquired for all cycles. Therefore fluorescence values are acquired while switching between plural amplification factors at each single cycle of the amplification reaction. In the example of Fig. 10 a fluorescence value amplified 1 times (not amplified), a fluorescence value amplified 1.8 times, a fluorescence value amplified 2.8 times and a fluorescence value amplified 5 times are respectively acquired at cycle 1. Then fluorescence values amplified 1, 1.8, 2.8 and 5 times are similarly respectively acquired in the second cycle. This is similarly repeated from cycle 3 onwards. By thus acquiring the amplified fluorescence values by switching between plural amplification factors every cycle, determination can be made that fluorescence values with a given amplification factor are no longer required at the point where a fluorescence value has reached a saturated amplification factor or a certain amount of change in the fluorescence values has been ascertained. Fluorescence values with this given amplification factor then no longer need to be acquired for subsequent cycles thereto. Thereby it is possible to accommodate plural amplification factors and also reduce the memory capacity required even in cases where it is difficult to predict the level of fluorescence values.

When the fluorescence values amplified by the largest amplification factor have exceeded the detection threshold value, by taking as detection results the fluorescence values amplified by the next largest amplification factor, detection results are obtained that efficiently utilize the gradations capable of being expressed by the apparatus. The fluorescence values can accordingly be detected with good precision.

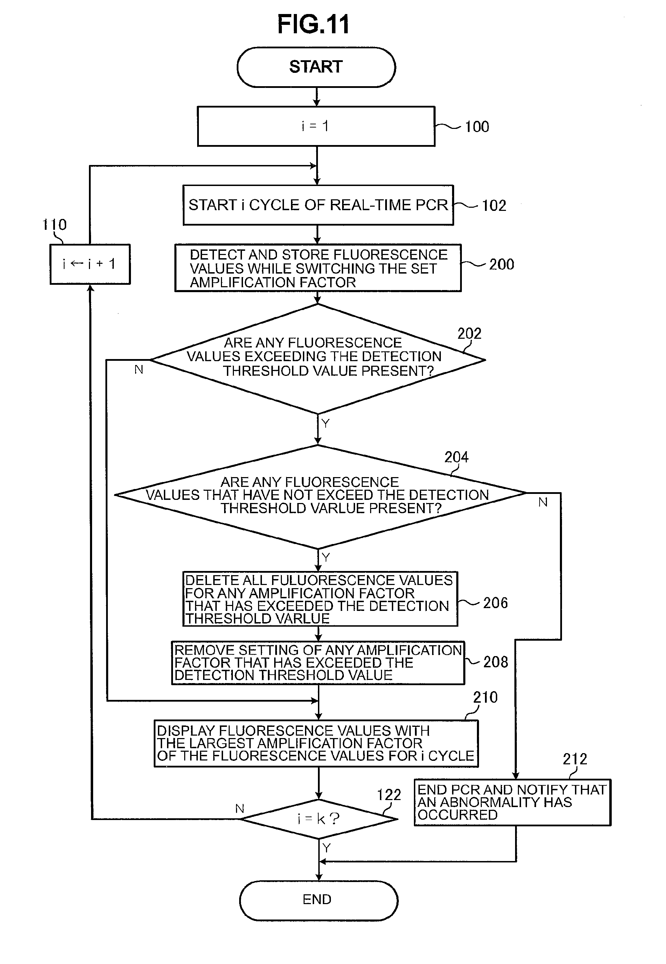

Explanation follows regarding operation of the nucleic acid detection apparatus 10 of the second exemplary embodiment. Explanation here is of a case in which amplification of target nucleic acid is performed in real-time PCR. First a PCR reaction liquid (sample) including a specimen and a reagent such as a probe, is placed in an analyzing section, not shown in the drawings. The probe employed here is a probe that emits fluorescence when hybridized to the region of the target sequence of the target nucleic acid, and has reduced fluorescence intensity when not hybridized to the target sequence region. Such cases result in a reaction system in which the level of the fluorescence values detected increases as the amplification reaction progresses. The nucleic acid detection processing routine illustrated in Fig. 11 is then executed by the CPU 30 by performing analysis start instruction from the display and operation section 24. Note that processing similar to that of the nucleic acid detection processing of the first exemplary embodiment is allocated the same reference numerals and more detailed explanation thereof is omitted.

At step 100 a variable i indicating the number of cycles of real-time PCR is set to 1, then at step 102 the i^{th} cycle of real-time PCR is started.

At the next step 200 the fluorescence value corresponding to the fluorescence intensity emitted in the PCR started at step 102 is detected. More specifically, similarly to in the first exemplary embodiment, excitation light is illuminated by the light source 12 onto the PCR reaction liquid placed in the analyzing section. Due to the excitation light, fluorescence is emitted according to the amount of target nucleic acid that has been amplified by PCR and is present in the PCR reaction liquid. When the emitted fluorescence is received by the light reception section 14, the light reception section 14 outputs an electrical signal at a level corresponding to the received fluorescence intensity. The output electrical signal is input respectively to the plural amplification circuits 16a to 16n that have different amplification factors from each other. The electrical signal is amplified here according to the amplification factor of each of the amplification circuits 16a to 16n and then output. The amplification factors here increase in the following sequence: amplification factor a of the amplification circuit 16a, amplification factor b of the amplification circuit 16b up to amplification factor n of the amplification circuit 16n.

The multiplexor 18 is input with a selection signal from the CPU 30 for switching amplification factors. The selected amplification factor(s) are set by the CPU 30 and stored in the ROM 32. All the amplification factors a, b, and so on to n are set in the initial settings. The selected electrical signal(s) are converted into digital signals by the A/D converter 20 and input to the computer 22. The levels of these electrical signals (the fluorescence values) are stored in the memory 36 against the cycle variable i and the respective selected amplification factor.

More specifically, the electrical signal output from the amplification circuit 16a with amplification factor a is first selected by inputting the selection signal for selecting the amplification factor a from the CPU 30 to the multiplexor 18. The selected electrical signal is converted to a digital signal by the A/D converter 20, input to the computer 22, and the level of this electrical signal (the fluorescence value) is stored in the memory 36 against the cycle number i and the amplification factor a. Then the selection signal for selecting the amplification factor b is input from the CPU 30 to the multiplexor 18, such that the electrical signal output from the amplification circuit 16b with the amplification factor b is selected, similarly to above. This fluorescence value is stored in the memory 36 against the cycle number i and the amplification factor b. This processing is repeated for each of the set amplification factors. Fluorescence values amplified by amplification factors a, b, and so on to n are accordingly acquired for cycle i.

In the following the fluorescence value amplified with amplification factor a in the i^{th} cycle is expressed as fluorescence value ia. The fluorescence values amplified by the amplification factors b to n are expressed similarly.

Then at step 202 determination is made as to whether or not fluorescence values that exceed the apparatus detection threshold value are present in the fluorescence values detected at step 220. When there are fluorescence values present that exceed the detection threshold value the routine transitions to step 204, and the routine transitions to step 210 when none are present. This example assumes that none of the fluorescence values have exceeded the detection threshold value and so the routine transitions to step 210.

At step 210 the largest of the fluorescence values detected in the i^{th} cycle (the fluorescence values detected in the immediately preceding step 200) is selected from the fluorescence values stored in the memory 36. The fluorescence values from the 1^{st} to the i^{th} cycles amplified with the amplification factor corresponding to the selected fluorescence value are then displayed on the display and operation section 24. In this case the fluorescence value 1a to fluorescence value ᵢₐ are displayed.

Then at step 122 determination is made as to whether or not the total number of cycles have been completed by determining whether or not the variable i indicating the cycle number has reached k. For example k may be set at 50 times. When i ≠ k processing transitions to step 110 since the total number of cycles has not yet been completed, the variable i is incremented by 1 and the routine returns to step 102 where processing is repeated for the next cycle of PCR.

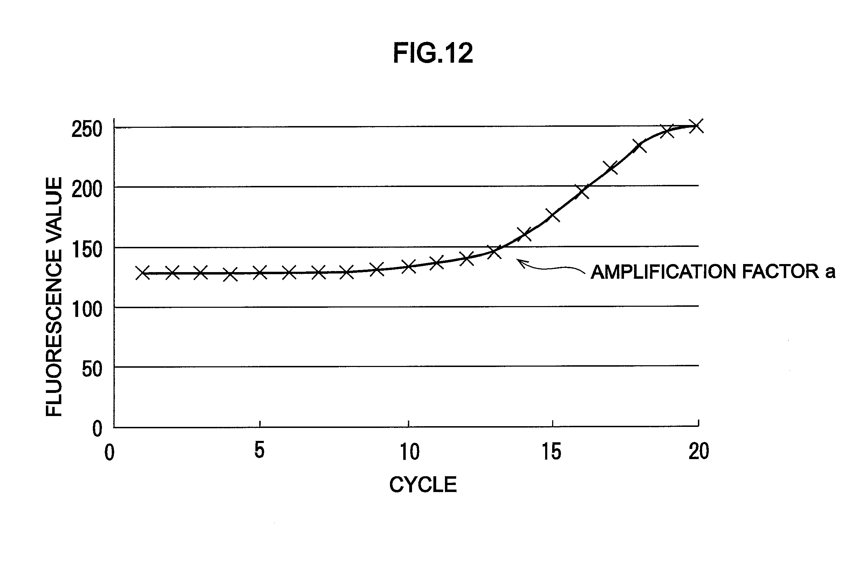

An example of detection results for display at step 210 is illustrated in Fig. 12 for a case in which none of the fluorescence values up to the 20^{th} cycle has exceeded the detection threshold value when the above processing is repeated. Since none of the fluorescence values up to the 20^{th} cycle has exceeded the detection threshold value the fluorescence value 1a to fluorescence value 20a that are amplified with the largest amplification factor a are displayed as the detection results. Then the routine transitions to step 204 in a case in which the fluorescence value 21a amplified by the amplification factor a exceeds the detection threshold value in the 21^{st} cycle.

At step 204 determination is made as to whether or not there are fluorescence values present in the fluorescence values detected at step 200, that do not exceed the detection threshold value. When such fluorescence values not exceeding the detection threshold value are present the routine transitions to step 206, however when none are present the routine transitions to step 212. In this example only the fluorescence value 21a amplified with the amplification factor a exceeds the detection threshold value, and the fluorescence values amplified with other amplification factors (fluorescence value 21b and so on to fluorescence value ₂₁ₙ) do not exceed the detection threshold value. The routine therefore transitions to step 206.

At step 206 all of the fluorescence value 1a to fluorescence value 20a from the 1^{st} to the i^{th} cycle that were amplified with the amplification factor a corresponding to the fluorescence value 21a that has been determined at step 202 to have exceeded the detection threshold value are deleted from the memory 36. Then at step 208 the setting for the amplification factor a is removed from settings for amplification factors that are stored in the ROM 32.

The fluorescence value 1a to fluorescence value 21a have already been deleted when the routine has transitioned through step 206 to step 210, and so the largest of the fluorescence values detected in the 21^{st} cycle in the fluorescence values stored in the memory 36 is the fluorescence value _{21b}. The fluorescence value 1b to fluorescence value 21b are accordingly displayed as the detection results on the display and operation section 24.

The routine then returns to step 102 through steps 122 and 110, and processing is repeated for the next cycle of PCR. At this stage in the next step 200, since the setting for the amplification factor a has been removed at step 208, the selection signal for selecting the amplification factor a is no longer input to the multiplexor 18 from the CPU 30, thereby stopping acquisition of the fluorescence values amplified by the amplification factor a.

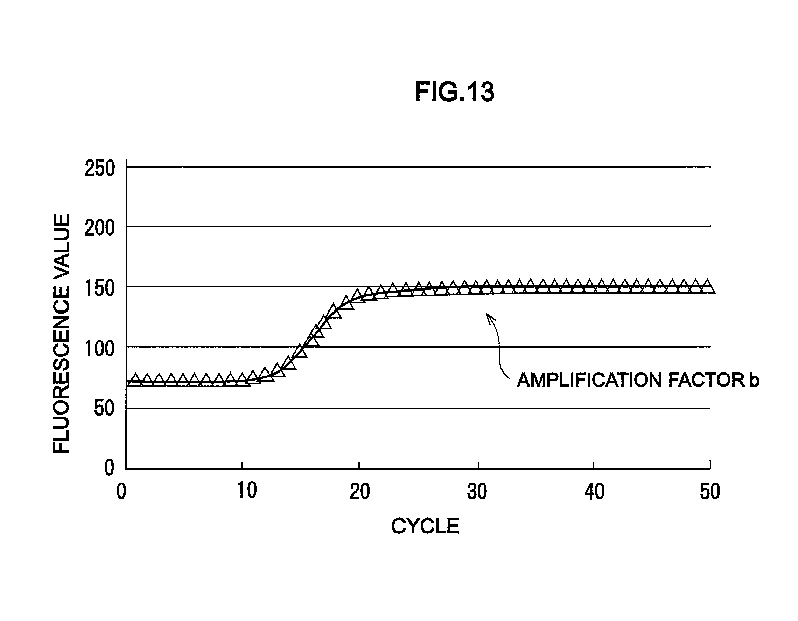

Processing is then ended when, due to repeating the above processing, it is determined at step 122 that i = k. Fig. 13 illustrates an example of detection results for displaying at step 210 when for example the fluorescence value 50b has not exceeded the detection threshold value in the 50^{th} cycle. Since the fluorescence values that were amplified with the largest amplification factor a exceeded the detection threshold value, the fluorescence value _{1b} to fluorescence value 50b amplified with the next largest amplification factor are displayed as the detection results.

However when it is determined at step 204 that there are no fluorescence values present that do not exceed the detection threshold value, the routine proceeds to step 121 and PCR is ended. Notification that an abnormality has occurred is made, such as by displaying on the display and operation section 24 a message indicating that fluorescence value acquisition failure has occurred, and processing is then ended.

As explained above, according to the nucleic acid detection apparatus of the second exemplary embodiment, the fluorescence values amplified by plural amplification factors are acquired while switching the amplification factor in each cycle. Application is accordingly possible in cases where it is difficult to predict the level of the fluorescence values that will be acquired and the memory capacity required can also be reduced. The fluorescence values amplified with the most appropriate amplification factors from the plural amplification factors, for example the largest of the fluorescence values that have not exceeded the apparatus detection threshold value, are displayed as detection results. The amount of the amplified target nucleic acid can accordingly be detected with good precision. Such an amplification factor can also be set automatically. Since a long time is required for the measurements themselves in amplification reactions and melting temperature measurements, even though a certain amount of time is required to switch the amplification factors this has limited impact on the overall time taken for measurement.

Explanation has been given of a case in the second exemplary embodiment in which an amplification reaction is performed by real-time PCR, however the present invention can also be applied to melting temperature measurements of target nucleic acid. In such cases configuration may be made such that plural fluorescence values are detected while switching amplification factors at each measurement point temperature (for example every 1°C) rather than for each cycle.

Explanation has been given of a case in the second exemplary embodiment in which all of the fluorescence values up to the current cycle that were amplified by an amplification factor corresponding to fluorescence value(s) that exceeded the apparatus detection threshold value are deleted, and fluorescence values amplified with these amplification factor(s) are no longer acquired in subsequent cycles, however there is no limitation thereto. For example, when there is spare memory capacity, configuration may be made only to stop acquisition of such fluorescence values in subsequent cycles, without deleting such fluorescence values that have already been acquired. Configuration may also be made such that, based on the fluorescence values detected at a specific cycle from the start of measurement, any fluorescence values of a specific threshold value or lower are deleted, and amplification factor(s) corresponding to the fluorescence value(s) of the specific threshold value or lower are removed from setting for subsequent cycles. A further reduction in required memory capacity can thereby be achieved.

Explanation has been given of a case in the second exemplary embodiment in which, when displaying detection results, a graph is plotted and displayed with the detected fluorescence values unmodified, however there is no limitation thereto. For example, discrete fluorescence values detected at each cycle may be corrected to give a smooth graph for display by performing smoothing and baseline correction.

Explanation has been given of a case in the second exemplary embodiment in which a probe is employed that emits fluorescence when hybridized to the target sequence region of the target nucleic acid and has reduced fluorescence intensity when not hybridized to the target sequence region, however there is no limitation thereto. A probe may be employed that emits fluorescence when not hybridized to the target sequence region of the target nucleic acid and has reduced fluorescence intensity when hybridized to the target sequence region.

Explanation has been given of a case in the second exemplary embodiment of a reaction system in which the fluorescence values increase as the amplification reaction progresses, however application may be made to a reaction system in which the fluorescence values reduce as amplification progresses.

Explanation has been given of cases in the first and the second exemplary embodiments wherein real-time PCR is employed for the amplification reaction, however there is no limitation thereto and for example a LAMP method, an invader method or RT-PCR may be employed. The light for detection is also not limited to fluorescence and depending on the type of sample and amplification reaction method detection may be made, for example, with light such as infrared light.

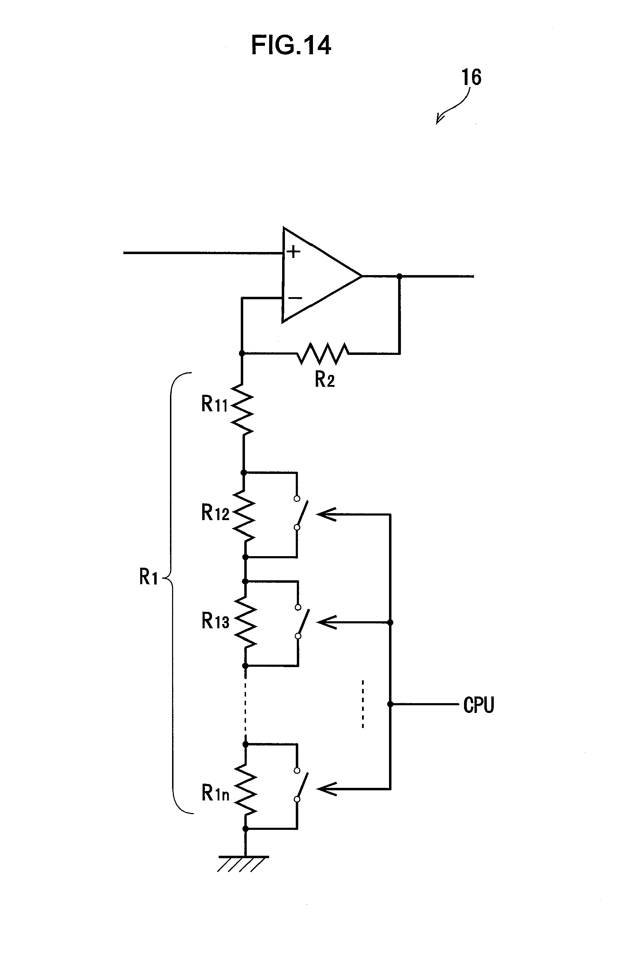

Moreover, explanation has been given of cases in the first and the second exemplary embodiments in which plural amplification circuits are provided having different amplification factors to each other, however configuration may be made with a single amplification circuit with plural switchable amplification factors. For example, configuration may be made as illustrated in Fig. 14 with a single amplification circuit 16. The amplification factors of the amplification circuit 16 are 1 + R1/R2, wherein R1 = R11 + R12 + R13 + and so on to R1n. Accordingly ON/OFF control may be performed using selection signals from a CPU to respective switches connected in parallel to each of the resistors R12, R13 and so on to R1n. The configuration of the combination of plural amplification circuits 16a to 16n of the first and the second exemplary embodiments is an example of an amplification unit of the present invention. The amplification circuit 16 in Fig. 14 is an example of the amplification unit of the present invention.

Explanation has been given of cases in the first and the second exemplary embodiments in which the detection results are displayed on the display and operation section. However, a printing device may be provided to the nucleic acid detection apparatus and the detection results printed out on a medium such as paper. The detection results may also be stored on a portable storage medium, or the detection results output to an external device connected through a network.

Note that a program that defines the above nucleic acid detection processing routine may be provided stored on a storage medium.

### Explanation of the Reference Numerals

- 10: NUCLEIC ACID DETECTION APPARATUS
- 12: LIGHT SOURCE
- 14: LIGHT RECEPTION SECTION
- 16, 16a to 16n: AMPLIFICATION CIRCUITS
- 18: MULTIPLEXOR
- 20: A/D CONVERTER
- 22: COMPUTER
- 24: DISPLAY AND OPERATION SECTION
- 30: CPU
- 32: ROM
- 34: RAM
- 36: MEMORY

## Claims

1. A nucleic acid detection apparatus, comprising:
a detection unit that detects an amount of an amplified or melted target nucleic acid at a plurality of points in time in an amplification reaction or melting temperature measurement of the target nucleic acid, by detection using an electrical signal whose level depends on light intensity emitted according to the amount of the target nucleic acid;
an amplification unit that amplifies an electrical signal detected by the detection unit with a specific amplification factor; and
a control unit that, based on an electrical signal detected by the detection unit in an initial stage of the amplification reaction or the melting temperature measurement, and based on an apparatus detection threshold value, effects control so as to change the amplification factor of an electrical signal detected during the amplification reaction or the melting temperature measurement after the initial stage.

2. The nucleic acid detection apparatus of claim 1, wherein the detection unit detects an electrical signal whose level decreases as the amplification reaction or the melting temperature measurement proceeds.

3. The nucleic acid detection apparatus of claim 1 or claim 2, wherein a probe capable of hybridizing to a target sequence region of the target nucleic acid is employed in the amplification reaction or the melting temperature measurement.

4. The nucleic acid detection apparatus of claim 3, wherein the probe emits fluorescence when not hybridized to the target sequence region and has reduced fluorescence intensity when hybridized to the target sequence region.

5. The nucleic acid detection apparatus of any one of claim 1 to claim 4, wherein the control unit performs at least one of the following controls when the level of the electrical signal detected by the detection unit in the initial stage exceeds a predetermined range:
control to provide notification that an abnormality has occurred in the amplification reaction or the melting temperature measurement; or
control to stop the amplification reaction or the melting temperature measurement or control to change the amplification factor.

6. A nucleic acid detection apparatus, comprising:
a detection unit that detects an amount of an amplified or melted target nucleic acid at a plurality of points in time in an amplification reaction or melting temperature measurement of the target nucleic acid, by detection using an electrical signal whose level depends on light intensity emitted according to the amount of the target nucleic acid;
an amplification unit that amplifies an electrical signal detected by the detection unit with a plurality of different amplification factors; and
a control unit that effects control at each of the plurality of points in time to switch an amplification factor of the amplification unit so as to acquire a plurality of electrical signals amplified by the respective plurality of different amplification factors.

7. The nucleic acid detection apparatus of claim 6, wherein the control unit effects control so as to store a electrical signal that is at a level of an apparatus detection threshold value or lower from among the plurality of acquired electrical signals.

8. The nucleic acid detection apparatus of claim 6 or claim 7, wherein the control unit effects control so as to display an electrical signal that is at a level of the apparatus detection threshold value or lower from among the plurality of acquired electrical signals, and to display the level at each point in time of electrical signals amplified with an amplification factor corresponding to an electrical signal with the largest value at the current point in time.

9. The nucleic acid detection apparatus of any one of claim 6 to claim 8, wherein the control unit effects control such that any electrical signal amplified with an amplification factor corresponding to an electrical signal that has exceeded the apparatus detection threshold value is not acquired at subsequent points in time.

10. The nucleic acid detection apparatus of any one of claim 6 to claim 9, wherein the control unit performs at least one of the following controls when all of the plurality of acquired electrical signals have exceeded the apparatus detection threshold value:
control to notify that an abnormality has occurred in the amplification reaction or the melting temperature measurement; or
control to stop the amplification reaction or the melting temperature measurement.

11. The nucleic acid detection apparatus of any one of claim 1 to claim 10, wherein the amplification reaction of the target nucleic acid comprises real-time PCR.

12. A nucleic acid detection method, comprising:
detecting an amount of an amplified or melted target nucleic acid, at a plurality of points in time in an amplification reaction or melting temperature measurement of the target nucleic acid, by detection using an electrical signal whose level depends on light intensity emitted according to the amount of the target nucleic acid;
amplifying a detected electrical signal with a specific amplification factor; and
based on an electrical signal detected in an initial stage of the amplification reaction or the melting temperature measurement, and based on an apparatus detection threshold value, changing the amplification factor of an electrical signal detected during the amplification reaction or the melting temperature measurement after the initial stage.

13. A nucleic acid detection method, comprising:
detecting an amount of an amplified or melted target nucleic acid, at a plurality of points in time in an amplification reaction or melting temperature measurement of the target nucleic acid, by detection using an electrical signal whose level depends on light intensity emitted according to the amount of the target nucleic acid;
amplifying a detected electrical signal with a plurality of different amplification factors; and
at each of the plurality of points in time, switching an amplification factor of an amplification unit for amplifying the detected electrical signal such that a plurality of electrical signals amplified respectively by the plurality of different amplification factors are acquired.

14. A nucleic acid detection program that causes a computer to function as a control unit for a system in which an amplification factor of an amplification unit is a specific amplification factor for amplifying an electrical signal detected by a detection unit, that detects an amount of an amplified or melted target nucleic acid, at a plurality of points in time in an amplification reaction or melting temperature measurement of the target nucleic acid, by detection using an electrical signal whose level depends on light intensity emitted according to the amount of the target nucleic acid, wherein, based on an electrical signal detected by the detection unit in an initial stage of the amplification reaction or the melting temperature measurement and based on an apparatus detection threshold value, the control unit effects control to so as to change the amplification factor of an electrical signal detected during the amplification reaction or the melting temperature measurement after the initial stage of the amplification reaction or the melting temperature measurement.

15. A nucleic acid detection program that causes a computer to function as a control unit that effects control so as to switch the amplification factor of an amplification unit employing a plurality of different amplification factors to amplify electrical signals detected by a detection unit, that detects an amount of an amplified or melted target nucleic acid, at a plurality of points in time in an amplification reaction or melting temperature measurement of the target nucleic acid, by detection using an electrical signal whose level depends on light intensity emitted according to the amount of the target nucleic acid, so as to acquire a plurality of electrical signals respectively amplified by the plurality of different amplification factors.
